# EUROPEAN PATENT APPLICATION

(11) **EP 3 842 539 A1**
(43) Date of publication of application: **30.06.2021**
(21) Application number: 20828248.3
(22) Date of filing: 03.04.2020
(51) Int. Cl.: C12P 19/26

(54) **METHOD FOR SYNTHESIZING LACTO-N-BIOSE**

(30) Priority: 31.10.2019 CN 201911055516
(71) Applicant: Yantai Huakang Biotech.Co., Ltd, Shandong 246006 (CN)
(72) Inventor: FANG, Xu, Shandong 264006 (CN); DU, Zhiqiang, Shandong 264006 (CN)
(74) Representative: ZHAOffice SPRL
(86) International application number: PCT/CN2020/083230
(87) International publication number: WO 2021/082352

(57) **Abstract**

The present invention provides a method for synthesizing lacto-*N*-biose and belongs to the technical field of bioengineering and oligosaccharide synthesis. The present invention uses a multi-enzyme catalytic system with good biological safety and wide application, and an ATP regeneration cycle system is introduced into a multi-enzyme reaction system, so that the synthesis of lacto-*N*-biose and the utilization rate of substrates are improved. The present invention provides a novel lacto-*N*-biose synthetic route, which lays a foundation for large-scale industrial production of lacto-*N*-biose and has important economic values and social benefits. At the same time, the synthetic method of the present invention is efficient, mild, simple, easy to operate, low in cost and suitable for industrial production, and has a high practical application value.

SEQUENCE LISTING

<110> YANTAI HUAKANG BIOTECH. CO., LTD.

<120> METHOD FOR SYNTHESIZING LACTO-N-BIOSE

<130>

<160> 3

<170> PatentIn version 3.3

<210> 1
<211> 382
<212> PRT
<213> Galk

<400> 1

Met Ser Leu Lys Glu Lys Thr Gln Ser Leu Phe Ala Asn Ala Phe Gly
1               5                   10                  15

Tyr Pro Ala Thr His Thr Ile Gln Ala Pro Gly Arg Val Asn Leu Ile
            20                  25                  30

Gly Glu His Thr Asp Tyr Asn Asp Gly Phe Val Leu Pro Cys Ala Ile
        35                  40                  45

Asp Tyr Gln Thr Val Ile Ser Cys Ala Pro Arg Asp Asp Arg Lys Val
    50                  55                  60

Arg Val Met Ala Ala Asp Tyr Glu Asn Gln Leu Asp Glu Phe Ser Leu
65                  70                  75                  80

Asp Ala Pro Ile Val Ala His Glu Asn Tyr Gln Trp Ala Asn Tyr Val
                85                  90                  95

Arg Gly Val Val Lys His Leu Gln Leu Arg Asn Asn Ser Phe Gly Gly
            100                 105                 110

Val Asp Met Val Ile Ser Gly Asn Val Pro Gln Gly Ala Gly Leu Ser
        115                 120                 125

Ser Ser Ala Ser Leu Glu Val Ala Val Gly Thr Val Leu Gln Gln Leu
    130                 135                 140

Tyr His Leu Pro Leu Asp Gly Ala Gln Ile Ala Leu Asn Gly Gln Glu
145                 150                 155                 160

Ala Glu Asn Gln Phe Val Gly Cys Asn Cys Gly Ile Met Asp Gln Leu
                165                 170                 175

Ile Ser Ala Leu Gly Lys Lys Asp His Ala Leu Leu Ile Asp Cys Arg
            180                 185                 190

Ser Leu Gly Thr Lys Ala Val Ser Met Pro Lys Gly Val Ala Val Val
        195                 200                 205

Ile Ile Asn Ser Asn Phe Lys Arg Thr Leu Val Gly Ser Glu Tyr Asn
    210                 215                 220

Thr Arg Arg Glu Gln Cys Glu Thr Gly Ala Arg Phe Phe Gln Gln Pro
225                 230                 235                 240

Ala Leu Arg Asp Val Thr Ile Glu Glu Phe Asn Ala Val Ala His Glu
                245                 250                 255

Leu Asp Pro Ile Val Ala Lys Arg Val Arg His Ile Leu Thr Glu Asn
            260                 265                 270

Ala Arg Thr Val Glu Ala Ala Ser Ala Leu Glu Gln Gly Asp Leu Lys
        275                 280                 285

Arg Met Gly Glu Leu Met Ala Glu Ser His Ala Ser Met Arg Asp Asp
    290                 295                 300

Phe Glu Ile Thr Val Pro Gln Ile Asp Thr Leu Val Glu Ile Val Lys
305                 310                 315                 320

Ala Val Ile Gly Asp Lys Gly Gly Val Arg Met Thr Gly Gly Gly Phe
                325                 330                 335

Gly Gly Cys Ile Val Ala Leu Ile Pro Glu Glu Leu Val Pro Ala Val
            340                 345                 350

Gln Gln Ala Val Ala Glu Gln Tyr Glu Ala Lys Thr Gly Ile Lys Glu
        355                 360                 365

Thr Phe Tyr Val Cys Lys Pro Ser Gln Gly Ala Gly Gln Cys
    370                 375                 380

<210> 2
<211> 751
<212> PRT
<213> LNBP

<400> 2

Met Thr Ser Thr Gly Arg Phe Thr Leu Pro Ser Glu Glu Asn Phe Ala
1               5                   10                  15

Glu Lys Thr Lys Glu Leu Ala Glu Leu Trp Gly Ala Asp Ala Ile Arg
            20                  25                  30

Asn Ser Asp Gly Thr His Leu Asp Glu Ser Val Leu Ala Leu Gly Lys
        35                  40                  45

Lys Ile Tyr Ser Ala Tyr Phe Pro Thr Arg Ala His Asn Glu Trp Ile
    50                  55                  60

Thr Leu His Met Asp Glu Thr Pro Gln Val Tyr Leu Leu Thr Gly Arg
65                  70                  75                  80

Val Leu Ala Glu Ala Asp Ile Val Asp Val Pro Leu Met Asp Gly Phe
                85                  90                  95

Phe Glu Glu Gln Leu Lys Pro Asn Arg Asp Ala Asp Pro His Lys Tyr
            100                 105                 110

Trp Glu Val Val Asp Arg Thr Thr Asn Glu Val Val Asp Ala Ser Leu
        115                 120                 125

Trp Thr Leu Asp Glu Asp Thr Asp Thr Val His Val Ser Gly Ala Thr
    130                 135                 140

Pro Met His Glu Tyr Thr Val Ser Phe Leu Ala Tyr Ile Ile Trp Asp
145                 150                 155                 160

Pro Val Glu Met Tyr Asn His Leu Thr Asn Gly Trp Gly Asp Lys Glu
                165                 170                 175

His Glu Ile Pro Phe Asp Ile Tyr His Pro Ala Thr Arg Lys Phe Val
            180                 185                 190

Phe Asp Thr Phe Glu Gln Trp Leu Lys Asp Asn Pro Gln Val Asp Val
        195                 200                 205

Val Arg Phe Thr Thr Phe Phe Tyr Gln Phe Thr Leu Leu Phe Asp Gln
    210                 215                 220

Lys Gln Arg Glu Lys Val Val Asp Trp Phe Gly Cys Ala Cys Thr Val
225                 230                 235                 240

Ser Pro Ala Ala Leu Asp Asp Phe Glu Lys Glu Tyr Gly Tyr Arg Leu
                245                 250                 255

Arg Pro Glu Asp Phe Val Asp Gly Gly Ala Tyr Asn Ser Ala Trp Arg
            260                 265                 270

Val Pro Arg Lys Ala Gln Arg Asp Trp Ile Asp Phe Leu Ser Gly Phe
        275                 280                 285

Val Arg Ala Asn Val Lys Lys Leu Ala Asp Met Ser His Glu Ala Gly
    290                 295                 300

Lys Glu Ala Met Met Phe Leu Gly Asp Gln Trp Ile Gly Thr Glu Pro
305                 310                 315                 320

Tyr Lys Asp Gly Phe Glu Asp Leu Gly Leu Asp Ala Val Val Gly Ser
                325                 330                 335

Ile Gly Asp Gly Thr Thr Thr Arg Met Ile Ala Asp Ile Pro Gly Val
            340                 345                 350

Lys Tyr Thr Glu Gly Arg Phe Leu Pro Tyr Phe Phe Pro Asp Thr Phe
        355                 360                 365

Tyr Glu Gly Asn Asp Pro Ser Ile Glu Gly Leu Asp Asn Trp Arg Lys
    370                 375                 380

Ala Arg Arg Ala Ile Leu Arg Ser Pro Ile Ser Arg Met Gly Tyr Gly
385                 390                 395                 400

Gly Tyr Leu Ser Leu Ala Ala Lys Phe Pro Lys Phe Val Asp Thr Val
                405                 410                 415

Thr His Ile Ala Asp Glu Phe Arg Asp Ile His Asp Arg Thr Gly Gly
            420                 425                 430

Val Ala Ala Glu Gly Glu Leu Asn Val Ala Ile Leu Asn Ser Trp Gly
        435                 440                 445

Lys Met Arg Ser Trp Met Ala Phe Thr Val Ala His Ala Leu Pro Asn
    450                 455                 460

Lys Gln Thr Tyr Ser Tyr Tyr Gly Ile Leu Glu Ser Leu Ser Gly Met
465                 470                 475                 480

Arg Val Asn Val Arg Phe Ile Ser Phe Asp Asp Val Leu Glu His Gly
                485                 490                 495

Val Ala Asp Asp Ile Asp Val Ile Ile Asn Gly Gly Pro Val Asp Thr
            500                 505                 510

Ala Phe Thr Gly Gly Asp Val Trp Lys Asn Pro Lys Leu Thr Glu Thr
        515                 520                 525

Leu Arg Ala Trp Val Arg Gly Gly Gly Ala Phe Val Gly Val Gly Glu
    530                 535                 540

Pro Ser Ser Leu Ala Arg Phe Gln Ala Gly Arg Phe Phe Gln Leu Ala
545                 550                 555                 560

Asp Val Leu Gly Val Asp Glu Glu Arg Tyr Gln Thr Leu Ser Val Asp
                565                 570                 575

Lys Tyr Phe Pro Thr Val Thr Pro Glu His Phe Ile Thr Ala Asp Val
            580                 585                 590

His Val Asp Pro Ala Ala Arg Glu Ala Trp Glu Lys Ala Gly Tyr Arg
        595                 600                 605

Ile Pro Leu Ser Gly Cys Gly Gly Gly Gln Gly Ile Lys Pro Leu Gly
    610                 615                 620

Gly Ile Asp Phe Gly Glu Pro Val Ala Asn Thr Phe Pro Val Asn Glu
625                 630                 635                 640

Asp Val Thr Leu Leu Arg Ala Asp Gly Gly Gln Val Gln Leu Ala Val
                645                 650                 655

Asn Glu Tyr Gly Lys Gly Arg Gly Val Tyr Ile Ser Gly Leu Pro Tyr
            660                 665                 670

Ser Ala Ala Asn Ala Arg Leu Leu Glu Arg Ala Leu Phe Trp Ala Ser
        675                 680                 685

His Asn Glu Asp Lys Tyr Thr Ala Tyr Ser Ser Thr Asn Pro Glu Cys
    690                 695                 700

Glu Val Ala Val Phe Pro Asp Ala Gly Gln Tyr Cys Val Ile Asn Asn
705                 710                 715                 720

Thr Asp Arg Pro Gln Ser Thr Asp Val Ala Leu Pro Asp Gly Ser Val
                725                 730                 735

Glu His Phe Asp Leu Asp Gln Ser Ala Ile Ala Trp Arg Asn Leu
            740                 745                 750

<210>  3
<211>  400
<212>  PRT
<213>  ACK

<400>  3

Met Ser Ser Lys Leu Val Leu Val Leu Asn Cys Gly Ser Ser Ser Leu
1               5                   10                  15

Lys Phe Ala Ile Ile Asp Ala Val Asn Gly Glu Glu Tyr Leu Ser Gly
            20                  25                  30

Leu Ala Glu Cys Phe His Leu Pro Glu Ala Arg Ile Lys Trp Lys Met
        35                  40                  45

Asp Gly Asn Lys Gln Glu Ala Ala Leu Gly Ala Gly Ala Ala His Ser
    50                  55                  60

Glu Ala Leu Asn Phe Ile Val Asn Thr Ile Leu Ala Gln Lys Pro Glu
65                  70                  75                  80

Leu Ser Ala Gln Leu Thr Ala Ile Gly His Arg Ile Val His Gly Gly
                85                  90                  95

Glu Lys Tyr Thr Ser Ser Val Val Ile Asp Glu Ser Val Ile Gln Gly
            100                 105                 110

Ile Lys Asp Ala Ala Ser Phe Ala Pro Leu His Asn Pro Ala His Leu
        115                 120                 125

Ile Gly Ile Glu Glu Ala Leu Lys Ser Phe Pro Gln Leu Lys Asp Lys
    130                 135                 140

Asn Val Ala Val Phe Asp Thr Ala Phe His Gln Thr Met Pro Glu Glu
145                 150                 155                 160

Ser Tyr Leu Tyr Ala Leu Pro Tyr Asn Leu Tyr Lys Glu His Gly Ile
                165                 170                 175

Arg Arg Tyr Gly Ala His Gly Thr Ser His Phe Tyr Val Thr Gln Glu
            180                 185                 190

Ala Ala Lys Met Leu Asn Lys Pro Val Glu Glu Leu Asn Ile Ile Thr
        195                 200                 205

Cys His Leu Gly Asn Gly Gly Ser Val Ser Ala Ile Arg Asn Gly Lys
    210                 215                 220

Cys Val Asp Thr Ser Met Gly Leu Thr Pro Leu Glu Gly Leu Val Met
225                 230                 235                 240

Gly Thr Arg Ser Gly Asp Ile Asp Pro Ala Ile Ile Phe His Leu His

245                 250                 255

Asp Thr Leu Gly Met Ser Val Asp Ala Ile Asn Lys Leu Leu Thr Lys
            260                 265                 270

Glu Ser Gly Leu Leu Gly Leu Thr Glu Val Thr Ser Asp Cys Arg Tyr
        275                 280                 285

Val Glu Asp Asn Tyr Ala Thr Lys Glu Asp Ala Lys Arg Ala Met Asp
    290                 295                 300

Val Tyr Cys His Arg Leu Ala Lys Tyr Ile Gly Ala Tyr Thr Ala Leu
305                 310                 315                 320

Met Asp Gly Arg Leu Asp Ala Val Val Phe Thr Gly Gly Ile Gly Glu
                325                 330                 335

Asn Ala Ala Met Val Arg Glu Leu Ser Leu Gly Lys Leu Gly Val Leu
            340                 345                 350

Gly Phe Glu Val Asp His Glu Arg Asn Leu Ala Ala Arg Phe Gly Lys
        355                 360                 365

Ser Gly Phe Ile Asn Lys Glu Gly Thr Arg Pro Ala Val Val Ile Pro
    370                 375                 380

Thr Asn Glu Glu Leu Val Ile Ala Gln Asp Ala Ser Arg Leu Thr Ala
385                 390                 395                 400

## Description

### BACKGROUND

### Technical Field

The present invention belongs to the technical field of bioengineering and oligosaccharide synthesis, and specifically relates to a method for synthesizing lacto-*N*-biose.

### Related Art

The background information is provided only to increase the understanding of the overall background of the present invention, but is not necessarily regarded as an acknowledgement or in any form suggesting that the information constitutes the prior art known to a person of ordinary skill in the art.

Breast milk, as the only material and energy link between the newborn and the mother after birth, is considered as the gold standard for infant nutrition. In addition to nutrients such as protein, fat and carbohydrates necessary for infant growth, what the breast milk provides also includes active components such as enzymes, antibodies, growth factors and oligosaccharides that can promote infant health and development. It is found that the biggest difference between cow and goat milk and breast milk lies in that breast milk is rich in functional human milk oligosaccharides (HMOs) and derivatives thereof with the content as high as 12-24 g/L, which are the third most important nutrient in breast milk. HMOs have special effects on the human body, especially for the perfection of the digestive system, intestinal health and immune system of infants and young children. Recently researchers found that HMOs can act as prebiotics for intestinal probiotics to regulate and promote the maturation of the intestinal immune barrier of infants, also can act as decoy molecules for pathogens, have antibacterial and antifungal effects, and protect infants from pathogen infection. Therefore, researches on HMOs are of great significance to the health of infants and young children, and addition of HMOs into milk powder to simulate breast milk components is particularly important for some infants who cannot be breast fed and the nutrition and health of infants after lactation.

However, due to limited availability and difficulty in large-scale synthesis with chemical methods, the research and wide application of biological functions of HMOs are restricted. In order to break through the bottleneck, it is necessary to establish a simple, efficient and economical synthetic route to obtain a large amount of the core structure lacto-*N*-biose (LNB, Galβ1-3GlcNAc, the chemical structural formula is as follows). Therefore, the low-cost, simple and easily separated technical process for biological synthesis of lacto-*N*-biose (LNB) was developed.

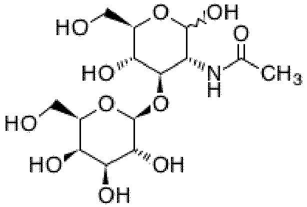

At present, there have been a number of patents focused on biosynthesis technologies for production of human milk oligosaccharides in *Escherichia coli,* such as:
patent name: "A fucosyltransferase and genetic engineering bacteria and application thereof" (CN201611147477.8),
patent name: "Production of human milk oligosaccharides in a microbial host with modified input/output" (CN201680052611.8),
patent name: "A fucosyltransferase and application thereof" (CN201611147478.2), and
patent name: "A method for preparing lacto-*N*-neotetraose (LNNT) containing N-acetyllactosamine" (CN201510751641.5).

In all the above-mentioned patents, a technology of using *Escherichia coli* as a host for heterologous production of human milk oligosaccharides is adopted. However, it is a great challenge to remove *Escherichia coli* endotoxin in large-scale industrial production. Endotoxin is a component in the cell wall of gram-negative bacteria, also known as lipopolysaccharide, which is a substance that is toxic to the human body. However, food safety of human milk oligosaccharides, especially as an additive for infant milk powder, is obviously very important. Therefore, it is particularly important to find a synthetic method suitable for large-scale industrial production of human milk oligosaccharides with good safety, stable yield and high production efficiency.

### SUMMARY

In view of the above-mentioned shortcomings of the prior art, the present invention uses a multi-enzyme catalytic system with good biological safety and wide application, and an ATP regeneration cycle system is introduced into a multi-enzyme reaction system, so that the synthesis of lacto-*N*-biose and the utilization rate of substrates are improved. The present invention provides a novel lacto-*N*-biose synthetic route, which lays a foundation for large-scale industrial production of lacto-*N*-biose and has important economic values and social benefits.

In the first aspect, the present invention provides a method for synthesizing lacto-*N-*biose, and the method comprises:
adding galactokinase (GalK) and lacto-*N*-biose phosphorylase (LNBP) into a reaction system containing galactose (Gal), acetylglucosamine (GlcNAc) and lactose (Lac) as substrates to prepare lacto-*N-*biose; and
adding acetyl phosphate and acetate kinase (ACK) into the above reaction system for in-situ regeneration of ATP.

Further, the method further comprises: separating the product lacto-*N*-biose and ATP and ADP present in the system.

The synthesis principle of the present invention is as follows: in the present invention, a multi-enzyme catalytic system using galactose as a substrate is constructed to synthesize lacto-*N-*biose. The reaction substrate, galactose, is catalyzed by galactokinase to produce galactose-1-phosphate, and ATP is degraded into ADP at the same time; further, galactose-1-phosphate produces lacto-*N*-biose under the action of lacto-*N*-biose phosphorylase (LNBP). Under the action of acetate kinase (ACK), ADP obtained above combines with acetyl phosphate to regenerate ATP, realizing regeneration of ATP. In the present invention, only a low concentration of ATP needs to be put into reactants, the reaction cost is greatly reduced, the conversion efficiency of substrates is improved, and the effect of inhibiting galactokinase by a high concentration of ATP is avoided, thereby achieving complete conversion of substrates and shortening the reaction time.

In the second aspect, the present invention provides lacto-*N*-biose prepared and synthesized by the synthetic method. In addition, based on the synthetic method of the present invention, the synthesis of all oligosaccharides or polysaccharides containing and/or using lacto-*N*-biose as a skeletal structure is also within the protection scope of the present invention.

### Beneficial technical effects of the present invention:

In the present invention, galactose is used as a raw material substrate, and an oligosaccharide structure catalytically synthesized in two steps includes lacto-*N-*biose. At the same time, all oligosaccharides or polysaccharides with lacto-*N*-biose as a skeletal structure can also be synthesized, the product preparation cost is low, and the practical value is high. In the present invention, the multi-enzyme catalytic system with good biological safety and wide application is used, and an ATP regeneration cycle system is introduced into the multi-enzyme reaction system, a simplified and economical coupling system for enzymatic synthesis of lacto-*N*-biose and regeneration of ATP is established, and the synthesis of lacto-*N*-biose and the utilization rate of substrates are improved.

In summary, the present invention provides a novel lacto-*N*-biose synthetic route, which lays a foundation for large-scale industrial production of lacto-*N*-biose and analogues thereof and has important economic values and social benefits. At the same time, the raw materials used in the present invention are easily available, the synthetic method is efficient, mild, simple, easy to operate, low in cost, environmentally friendly and suitable for industrial production, and has a high practical application value.

### BRIEF DESCRIPTION OF THE DRAWINGS

The accompanying drawings constituting a part of the present invention are used to provide a further understanding of the present invention. The exemplary examples of the present invention and descriptions thereof are used to explain the present invention, and do not constitute an improper limitation of the present invention.
Figure 1 is a schematic diagram showing catalytic production of lacto-*N*-biose in two steps with galactose as a substrate of the present invention.
Figure 2 is a synthetic comparison diagram of lacto-*N*-biose at an initial amount of 5 mM ATP after introduction of ATP regeneration cycle in Example 2 of the present invention.
Figure 3 is a synthetic comparison diagram of lacto-*N*-biose at an initial amount of 7.5 mM ATP after introduction of ATP regeneration cycle in Example 3 of the present invention.

### DETAILED DESCRIPTION

It should be noted that the following detailed descriptions are all exemplary and are intended to provide a further description of the present invention. Unless otherwise specified, all technical and scientific terms used herein have the same meaning as commonly understood by those of ordinary skill in the art to which the present invention belongs.

It should be noted that terms used herein are only for the purpose of describing specific implementations and are not intended to limit the exemplary implementations of the present invention. As used herein, the singular form is intended to include the plural form, unless the context clearly indicates otherwise. In addition, it should be further understood that terms "comprise" and/or "include" used in this specification indicate that there are features, steps, operations, devices, assemblies, and/or combinations thereof. It should be understood that the protection scope of the present invention is not limited to the following specific implementation solutions. It should further be understood that terms used in the embodiment of this application are only for describing the specific implementation solutions, and are not intended to limit the protection scope of the present invention. In the following specific embodiments, if specific conditions are not indicated, experimental methods usually follow conventional methods and conditions of molecular biology in the art, and such techniques and conditions are fully explained in the literature. For example, Sambrook et al., techniques and conditions in "Molecular Cloning: Laboratory Manual" or conditions recommended by manufacturers are taken as references.

As mentioned earlier, it is currently difficult to synthesize human milk oligosaccharides on a large scale using chemical methods, and when a biotechnology is used for heterogeneous production of human milk oligosaccharides, the production efficiency is generally low, and food safety problems are likely to be caused.

In view of this, in a specific embodiment of the present invention, a method for synthesizing lacto-*N*-biose is provided and includes:
adding galactokinase and lacto-*N*-biose phosphorylase into a reaction system containing galactose, acetylglucosamine and lactose as substrates to prepare lacto-*N*-biose; and
adding acetyl phosphate and acetate kinase into the above reaction system for in-situ regeneration of ATP. The present invention aims at consuming one molecule of ATP energy in a galactokinase catalytic step, and constructs but is not limited to realization of ATP energy regeneration in acetyl phosphate with acetate kinase. Experimental results of the present invention prove that by introducing energy regeneration cycle, regeneration cycle of ATP and full conversion of substrates can be realized, thereby effectively improving the synthesis efficiency of lacto-*N*-biose.

In another specific embodiment of the present invention, the method further includes: separating the product lacto-*N*-biose and ATP and ADP present in the reaction system.

In the present invention, sources of enzymes used are not particularly limited, which may be extracted from natural bacteria, yeast or fungi and other microorganisms, produced by genetically engineered bacteria through genetic recombination and extracted from natural plant tissues or animal tissues. The enzyme product forms are also not particularly limited, which may be solid, powder, liquid, or an immobilized enzyme fixed on a carrier by a physical or chemical method. Enzymes may be commercially available products, or self-made products by enterprises or individuals.

In another specific embodiment of the present invention, the galactokinase, lacto-*N*-biose phosphorylase (LNBP) and acetate kinase are all produced by genetically engineered bacteria through genetic recombination.

Specifically, the method of the production includes: cloning expression vectors derived from galactokinase, lacto-*N*-biose phosphorylase and acetate kinase respectively; and obtaining target enzyme proteins by culturing and inducing hosts of the corresponding expression vectors.

In another specific embodiment of the present invention, the expression vectors are any one or more of viral vectors, plasmids, phages, phagemids, cosmids, F cosmids, bacteriophages or artificial chromosomes; viral vectors may include adenovirus vectors, retroviral vectors or adeno-associated virus vectors, and artificial chromosomes include bacterial artificial chromosomes (BAC), phage P1 derived carriers (PAC), yeast artificial chromosomes (YAC) or mammalian artificial chromosomes (MAC); further preferably, the expression vectors are plasmids; more preferably, the expression vectors are pET-28a plasmids.

In another specific embodiment of the present invention, the hosts include but are not limited to bacteria, fungi and eukaryotic cells, and are further selected from *Escherichia coli, Bacillus, Bacillus subtilis, Saccharomyces cerevisiae, Trichoderma reesei* and *Penicillium oxalicum;* more preferably, the hosts are *Escherichia coli* BL21 (DE3).

In another specific embodiment of the present invention, the method of the production includes: cloning expression plasmids pET28a-galk, pET28a-Lnbp and pET28a-ack derived from galactokinase, LNBP and acetate kinase respectively; and obtaining target enzyme proteins by culturing and inducing BL21(DE3) strains of the corresponding expression plasmids and carrying out purification (preferably by a nickel column).

Galactokinase source strains include but are not limited to *Escherichia coli;* LNBP source strains include but are not limited to *Bifidobacterium;* and ACK source strains include but are not limited to *Escherichia coli.*

In another specific embodiment of the present invention,
the amino acid sequence of galactokinase is shown as SEQ ID No. 1;
the amino acid sequence of LNBP is shown as SEQ ID No. 2; and
the amino acid sequence of acetate kinase is shown as SEQ ID No. 3.

In another specific embodiment of the present invention, the reaction system further contains ATP, and further, the concentration of ATP is 5-15 mM (preferably 7.5 mM).

The reaction system further contains magnesium ions (preferably MgCl₂) and a Tris-HCl buffer.

In another specific embodiment of the present invention, the concentration of MgCl₂ is 1-10 mM (preferably 3 mM); and the concentration of the Tris-HCl buffer is 10-200 mM (preferably 100 mM).

In another specific embodiment of the present invention, the reaction temperature of the reaction system is 25-45°C, and the reaction pH is 5.8-7.5.

In another specific embodiment of the present invention, the concentrations of the galactose and acetylglucosamine substrates are both 10-20 mM.

In another specific embodiment of the present invention, the concentration of galactokinase is 1-10 U/mL.

In another specific embodiment of the present invention, the enzyme concentration of LNBP is 100-300 U/mL.

In another specific embodiment of the present invention, the concentration of acetate kinase required for in-situ regeneration of ATP is 1-10 U/mL, and the concentration of acetyl phosphate is 2.5-5 mM.

In another specific embodiment of the present invention, lacto-*N*-biose synthesized by the synthetic method is provided. In addition, based on the synthetic method of the present invention, the synthesis of all oligosaccharides or polysaccharides with lacto-*N*-biose as a skeletal structure is also within the protection scope of the present invention.

The following further explains and describes the present invention through specific embodiments, but does not constitute a limitation on the present invention. It should be understood that these embodiments are only for illustrating the present invention and are not intended to limit the scope of the present invention. An experimental method without indicating a specific condition in the following embodiments is generally performed according to a conventional condition.

### Example 1: Synthesis of lacto-N-biose by using galactose as a substrate

10 mM galactose, 10 mM ATP, 10 mM GlcNAc, 3 mM MgCl₂, 100 mM Tris-HCl, 5 U Galk and 168 U lacto-*N*-biose phosphorylase are added into a 1 mL reaction system. As shown in Table 1, Control is a control group without addition of lacto-*N*-biose phosphorylase (LNBP). After a reaction at 37°C for 12 hours, the reaction system is boiled for 5 minutes and centrifuged, a supernatant is sampled, filtered through a filtering membrane and detected through a Biorad-HPX column, RID detection of corresponding substrates and products is carried out, reaction detection map (HPLC) comparison results verify the synthesis of lacto-*N*-biose.

**Table 1 A reaction table showing catalytic production of lacto-N-biose in two steps with galactose as a substrate**

| | Gal mM | ATP mM | GalK U/mL | MgCl₂ mM | LNBP U/mL | GlcNAc mM |
|---|---|---|---|---|---|---|
| Control | 10 | 10 | 5 | 3 | | 10 |
| 1 | 10 | 10 | 5 | 3 | 168 | 10 |

### Example 2: Synthesis of lacto-N-biose by introducing ATP regeneration cycle

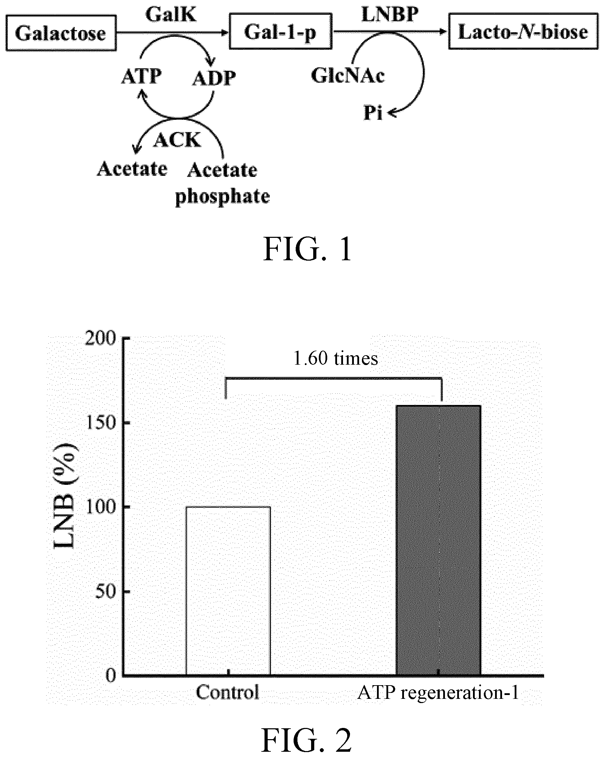

10 mM galactose, 7.5 mM ATP, 2.5 mM acetyl phosphate, 10 mM GlcNAc, 3 mM MgCl₂, 100 mM Tris-HCl, 5 U Galk, 3 U ACK and 168 U LNBP are added into a 1 mL reaction system. As shown in Table 2, the initial concentration of ATP in Control is 10 mM, and an ATP cyclic reaction is not introduced into the reaction system. After a reaction at 37°C for 12 hours, the reaction system is boiled for 5 minutes and centrifuged, a supernatant is sampled, filtered through a filtering membrane and detected by a Biorad-HPX column, and RID detection of corresponding substrates and products is carried out. As shown in Figure 2, under ATP-1 reaction conditions, the synthesis of LNB is increased by 1.60 times in comparison with the control group (without addition of ATP).

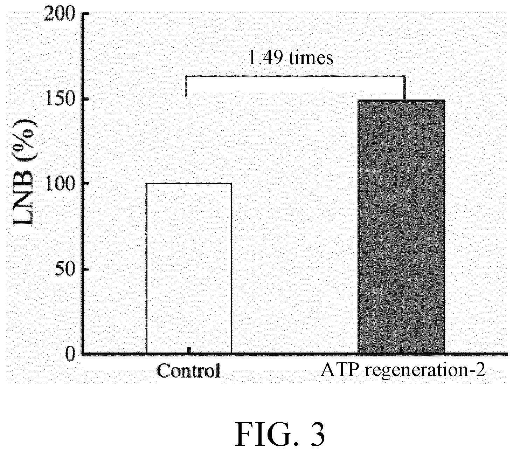

**Example 3:** 10 mM galactose, 5 mM ATP, 5 mM acetyl phosphate, 10 mM GlcNAc, 3 mM MgCl₂, 100 mM Tris-HCl, 5 U Galk, 3 U ACK and 168 U LNBP are added into a 1 mL reaction system. As shown in Table 2, the initial concentration of ATP in Control is 10 mM, and an ATP cyclic reaction is not introduced into the reaction system. After a reaction at 37°C for 12 hours, the reaction system is boiled for 5 minutes and centrifuged, a supernatant is sampled, filtered through a filtering membrane and detected by a Biorad-HPX column, and RID detection of corresponding substrates and products is carried out. As shown in Figure 3, under ATP-2 reaction conditions, the synthesis of LNB is increased by 1.49 times in comparison with the control group (without addition of ATP).

**Table 2 A reaction table showing catalytic production of lacto-N-biose in two steps with galactose as a substrate after introduction of ATP regeneration cycle**

| Reaction | Gal mM | ATP mM | GalK U | MgCl₂ mM | LNBP U | GlcNAc mM | Acetyl-ACP | ACK U |
|---|---|---|---|---|---|---|---|---|
| Control | 10 | 10 | 5 | 3 | 168 | 10 | | |
| ATP-1 | 10 | 7.5 | 5 | 3 | 168 | 10 | 2.5 | 3 |
| ATP-2 | 10 | 5 | 5 | 3 | 168 | 10 | 5 | 3 |

It should be noted that the foregoing embodiments are merely intended for describing the technical solutions of the present invention other than limiting the present invention. Although the present invention is described in detail with reference to the foregoing embodiments, a person skilled in the art may make modifications or equivalent substitutions to the technical solutions of the present invention as required, without departing from spirit and scope of the technical solutions of the present invention.

## Claims

1. A method for synthesizing lacto-*N*-biose, comprising:
adding galactokinase and lacto-*N*-biose phosphorylase into a reaction system containing galactose, acetylglucosamine and lactose as substrates to prepare lacto-*N*-biose; and
adding acetyl phosphate and acetate kinase into the above reaction system for in-situ regeneration of ATP.

2. The method for synthesizing lacto-*N*-biose according to claim 1, wherein the method further comprises: separating the product lacto-*N*-biose and ATP and ADP present in the reaction system.

3. The method for synthesizing lacto-*N*-biose according to claim 1, wherein the galactokinase, lacto-*N*-biose phosphorylase and acetate kinase are all produced by genetically engineered bacteria through genetic recombination.

4. The method for synthesizing lacto-*N*-biose according to claim 3, wherein the method of the production comprises: cloning expression vectors derived from galactokinase, lacto-*N*-biose phosphorylase and acetate kinase respectively; and obtaining target enzyme proteins by culturing and inducing hosts of the corresponding expression vectors.

5. The method for synthesizing lacto-*N*-biose according to claim 1, wherein
the amino acid sequence of galactokinase is shown as SEQ ID No. 1;
the amino acid sequence of lacto-*N*-biose phosphorylase is shown as SEQ ID No. 2; and
the amino acid sequence of acetate kinase is shown as SEQ ID No. 3.

6. The method for synthesizing lacto-*N*-biose according to claim 1, wherein the reaction system further contains ATP, and the concentration of ATP is 5-15 mM.

7. The method for synthesizing lacto-*N*-biose according to claim 6, wherein the concentration of ATP is 7.5 mM.

8. The method for synthesizing lacto-*N*-biose according to claim 1, wherein the reaction system further contains MgCl₂ and a Tris-HCl buffer;
the concentration of MgCl₂ is 1-10 mM; and the concentration of the Tris-HCl buffer is 10-200 mM.

9. The method for synthesizing lacto-*N*-biose according to claim 8, wherein the concentration of MgCl₂ is 3 mM, and the concentration of the Tris-HCl buffer is 100 mM.

10. The method for synthesizing lacto-*N*-biose according to claim 1, wherein the reaction temperature of the reaction system is 25-45°C, and the reaction pH is 5.8-7.5.

11. The method for synthesizing lacto-*N*-biose according to claim 1, wherein the concentrations of the galactose and acetylglucosamine substrates are both 10-20 mM;
the concentration of galactokinase is 1-10 U/mL;
the enzyme concentration of LNBP is 100-300 U/mL;
the concentration of acetate kinase is 1-10 U/mL; and
the concentration of acetyl phosphate is 2.5-5 mM.

12. Lacto-*N-*biose synthesized by the method according to any one of claims 1 to 11.
